# EUROPEAN PATENT SPECIFICATION

(11) **EP 3 199 162 B1**
(45) Date of publication and mention of the grant of the patent: **09.03.2022**
(21) Application number: 15844883.7
(22) Date of filing: 25.09.2015
(51) Int. Cl.: A61K 31/551, A61K 9/08, A61P 27/02, A61P 27/06, A61P 43/00

(54) **PHARMACEUTICAL PRODUCT**
PHARMAZEUTISCHES PRODUKT
PRODUIT PHARMACEUTIQUE

(30) Priority: 25.09.2014 JP 2014194965
(43) Date of publication of application: 02.08.2017
(73) Proprietor: Kowa Company, Ltd., Nagoya-shi, Aichi 460-8625 (JP)
(72) Inventor: SUGIMOTO, Shin, Fuji-shi Shizuoka 4178650 (JP)
(74) Representative: Blodig, Wolfgang
(86) International application number: PCT/JP2015/077014
(87) International publication number: WO 2016/047720

(56) References cited:
- EP-A1- 1 726 306
- EP-A1- 2 319 514
- EP-A1- 3 199 161
- EP-A1- 3 231 430
- WO-A1-2015/156321
- JP-A- 2006 290 827
- US-A1- 2008 064 681
- US-A1- 2008 064 681
- ISOBE, TOMOYUKI ET AL.: 'Effects of K-115, a Rho-Kinase Inhibitor, on Aqueous Humor Dynamics in Rabbits' CURRENT EYE RESEARCH vol. 39, no. 8, pages 813 - 822, XP009501175
- 'Tengan'yaku - Joshiki to Hijoshiki' GANKA NEW INSIGHT vol. 2, 10 July 1987, pages 15 - 23, XP009501405
- IYAKUHIN INTERVIEW FORM GLANATEC(R) TENGAN'EKI 0.4% June 2015, XP009501344
- ISOBE TOMOYUKI ET AL: "Effects of K-115, a Rho-Kinase Inhibitor, on Aqueous Humor Dynamics in Rabbits", CURRENT EYE RESEARCH, INFORMA HEALTHCARE USA, US, vol. 39, no. 8, 1 August 2014 (2014-08-01) , pages 813-822, XP009501175, ISSN: 0271-3683, DOI: 10.3109/02713683.2013.874444
- James Swarbrick: "Volume 1 21 CFR Part 11 Revisited / 1 Absorption Enhancers / 13 Absorption of Drugs / 19 Adsorption at Solid Surfaces: Pharmaceutical Applications / 34 Adverse Drug Reactions / 46 Advertising and Promotion of Prescription and Over-the-Counter Drug Products / 57 Alternative Medicines / 66 Amorphous P", , 1 January 2007 (2007-01-01), XP055649126, Retrieved from the Internet: URL:https://gmpua.com/Process/Encyclopedia PT.pdf [retrieved on 2019-12-04]

## Description

### [Field of the Invention]

The present invention relates to a pharmaceutical preparation and the like.

### [Background Art]

It is known that halogenated isoquinoline derivatives such as ripasudil (chemical name: 4-fluoro-5-[[(2S)-2-methyl-1,4-diazepan-1-yl]sulfonyl]isoquinoline) represented by the following structural formula: 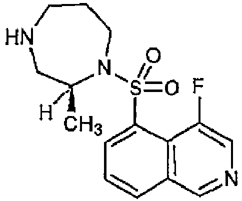 and 4-bromo-5-[[(2S)-2-methyl-1,4-diazepan-1-yl]sulfonyl]isoquinoline represented by the following structural formula: 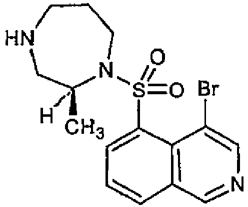 have pharmacological action such as Rho kinase inhibitory action (Patent Literatures 1 and 2, for example), and thus, are usable for the prevention or treatment of eye diseases. Specifically, these halogenated isoquinoline derivatives have been reported to be useful, for example, for the prevention or treatment of ocular hypertension, glaucoma, and the like (Patent Literature 3, for example), or for the prevention or treatment of ocular fundus diseases such as age-related macular degeneration and the like (Patent Literature 4, for example).

Hence, it is extremely useful to establish a technique for producing stable preparations of these halogenated isoquinoline derivatives as ophthalmic agents, for example.

### [Citation List]

### [Patent Literature]

[Patent Literature 1] JP-B-4212149
[Patent Literature 2] WO2006/115244
[Patent Literature 3] WO2006/068208
[Patent Literature 4] JP-B-5557408
Patent EP-1726306 discloses an ophthalmic agent containing 1-(5-Isoquinolinesulfonyl) homopiperazine hydrochloride or "fasudil hydrochloride" in an aqueous eye drop filled in brown glass container. The pH of the aqueous solution is 5.5 or less.Encyclopedia of Pharmaceutical Technology, DOI: 10.1081/E-EPT-100000368, 2007, p.1266-1278, 'Drug Delivery: parenteral Route' by M. Akers, this article proposes that ophthalmic solutions and suspensions are usually packaged in squeezable low-density polyethylene containers for easy administration.

### [Summary of Invention]

### [Technical Problem]

An ophthalmic agent or the like is generally a composition containing water (aqueous composition), and needs to be housed in a container to prevent dissipation of the contents or the inclusion of impurities from outside air, for example. Thus, to produce a preparation of a halogenated isoquinoline derivative, ripasudil, as an ophthalmic agent or the like, the present inventor studied materials of the container for storing the aqueous composition. As a result, the inventor found that housing an aqueous composition containing ripasudil in a container made of a specific material results in the problem of discoloration of the aqueous composition due to preservation at high temperature for a long period of time.

Accordingly, it is an object of the present invention to provide a technique for suppressing discoloration of an aqueous composition containing a halogenated isoquinoline derivative during preservation at high temperature.

### [Solution to Problem]

The present inventor thus conducted extensive research to solve the above-described problem, and found that when an aqueous composition containing a halogenated isoquinoline derivative such as ripasudil is stored in a container, the use of polyolefin-based resin as a material of the container can specifically suppress discoloration even after preservation at high temperature for a long period of time, thus completing the present invention.

Specifically, the present invention provides a pharmaceutical preparation which contains an aqueous composition comprising a compound represented by Formula (1) : 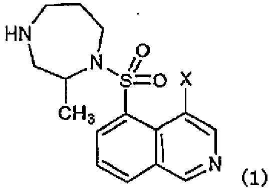 wherein X represents a halogen atom,
or a salt thereof, or a solvate of the compound or the salt thereof, is stored in a container made of polyolefin-based resin, wherein the polyolefin-based resin is polypropylene.

The present invention also provides the use of a container made of polyolefin-based resin, wherein the polyolefin-based resin is polyethylene or polypropylene, for suppressing discoloration of an aqueous composition comprising a compound represented by Formula (1), wherein X represents a halogen atom, or salt thereof, or a solvate of the compound or the salt thereof.

### [Effect of Invention]

In accordance with the present invention, discoloration of an aqueous composition containing a halogenated isoquinoline derivative such as ripasudil during preservation at high temperature can be suppressed.

### [Description of Embodiments]

The present specification discloses, although is in no way limited to, the following embodiments of invention, by way of example.
[1] A pharmaceutical preparation obtained by storing an aqueous composition comprising a compound represented by Formula (1): 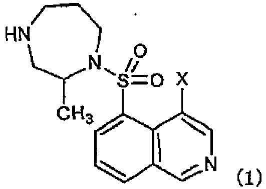 wherein X represents a halogen atom,
   or a salt thereof, or a solvate of the compound or the salt thereof, is stored in a container made of polyolefin-based resin, wherein the polyolefin-based resin is polypropylene.
[2] The pharmaceutical preparation according to [1], wherein the compound represented by Formula (1) is ripasudil.
[3] The pharmaceutical preparation according to [1] or [2], wherein the polyolefin-based resin is polypropylene.
[4] The pharmaceutical preparation according to any of [1] to [3], wherein the container made of polyolefin-based resin is a container for eye drops.
[5] A method for suppressing discoloration of an aqueous composition comprising the step of storing an aqueous composition comprising a compound represented by Formula (1), or a salt thereof, or a solvate of the compound or the salt thereof, in a container made of polyolefin-based resin.
[6] The method according to [5], wherein the compound represented by Formula (1) is ripasudil.
[7] The method according to [5] or [6], wherein the polyolefin-based resin is polyethylene or polypropylene.
[8] The method according to any of [5] to [7], wherein the container made of polyolefin-based resin is a container for eye drops.
[9] The pharmaceutical preparation according to any of [1] to [4], wherein the aqueous composition further contains one or more selected from the group consisting of α1 receptor blockers, α2 receptor agonists, β blockers, carbonic anhydrase inhibitors, prostaglandin F2α derivatives, sympathomimetics, parasympathomimetics, calcium antagonists, and cholinesterase inhibitors.
[10] The pharmaceutical preparation according to any of [1] to [4], wherein the aqueous composition further contains one or more selected from the group consisting of latanoprost, nipradilol, dorzolamide, brinzolamide, and timolol, as well as salts thereof.
[11] The method according to any of [5] to [8], wherein the aqueous composition further contains one or more selected from the group consisting of α1 receptor blockers, α2 receptor agonists, β blockers, carbonic anhydrase inhibitors, prostaglandin F2α derivatives, sympathomimetics, parasympathomimetics, calcium antagonists, and cholinesterase inhibitors.
[12] The method according to any of [5] to [8], wherein the aqueous composition further contains one or more selected from the group consisting of latanoprost, nipradilol, dorzolamide, brinzolamide, and timolol, as well as salts thereof.

Examples of the halogen atom in Formula (1) include a fluorine atom, a chlorine atom, and a bromine atom. In Formula (1), a fluorine atom or a bromine atom is preferred as the halogen atom, and a fluorine atom is particularly preferred.

Further, in Formula (1), the carbon atom forming the homopiperazine ring substituted with the methyl group is an asymmetric carbon atom. As a result, stereoisomerism occurs. The compound represented by Formula (1) includes all the stereoisomers, and may be a single stereoisomer or a mixture of various stereoisomers at any given ratio. Preferred as the compound represented by Formula (1) is a compound having the S configuration as the absolute configuration.

The salt of the compound represented by Formula (1) is not particularly limited as long as it is a pharmacologically acceptable salt, and specific examples of the salt include inorganic acid salts such as hydrochloride, sulfate, nitrate, hydrofluoride, and hydrobromate; and organic acid salts such as acetate, tartrate, lactate, citrate, fumarate, maleate, succinate, methanesulfonate, ethanesulfonate, benzenesulfonate, toluenesulfonate, naphthalenesulfonate, and camphorsulfonate, with hydrochloride being preferred.

The compound represented by Formula (1) or a salt thereof may also be in the form of a hydrate or a solvate such as an alcohol solvate, and is preferably in the form of a hydrate.

Specific examples of the compound represented by Formula (1), or a salt thereof, or a solvate of the compound or the salt thereof, include:
ripasudil (chemical name: 4-fluoro-5-[[(2S)-2-methyl-1,4-diazepan-1-yl]sulfonyl]isoquinoline), or a salt thereof, or a solvate of ripasudil or the salt thereof; and
4-bromo-5-[[(2S)-2-methyl-1,4-diazepan-1-yl]sulfonyl]isoquinoline, or a salt thereof, or a solvate of 4-bromo-5-[[(2S)-2-methyl-1,4-diazepan-1-yl]sulfonyl]isoquinoline or the salt thereof.

The compound represented by Formula (1), or a salt thereof, or a solvate of the compound or the salt thereof, is preferably ripasudil, or a salt thereof, or a solvate of ripasudil or the salt thereof, or 4-bromo-5-[[(2S)-2-methyl-1,4-diazepan-1-yl]sulfonyl]isoquinoline, or a salt thereof or a solvate of 4-bromo-5-[[(2S)-2-methyl-1,4-diazepan-1-yl]sulfonyl]isoquinoline or the salt thereof, more preferably ripasudil, or a salt thereof, or a solvate of ripasudil or the salt thereof, even more preferably ripasudil or a hydrochloride thereof, or a hydrate of ripasudil or the hydrochloride thereof, and particularly preferably a ripasudil hydrochloride hydrate (ripasudil monohydrochloride dihydrate) represented by the following structural formula:

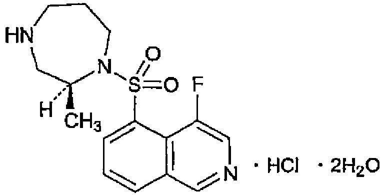

The compound represented by Formula (1), or a salt thereof, or a solvate of the compound or the salt thereof is known and can be produced using a known method. Specifically, ripasudil, or a salt thereof, or a solvate of ripasudil or the salt thereof, can be produced using the method described in WO1999/020620 or WO2006/057397, for example. 4-Bromo-5-[[(2S)-2-methyl-1,4-diazepan-1-yl]sulfonyl]isoquinoline, or a salt thereof, or a solvate of 4-bromo-5-[[(2S)-2-methyl-1,4-diazepan-1-yl]sulfonyl]isoquinoline or the salt thereof can be produced using the method described in WO2006/115244, for example.

The content of the compound represented by Formula (1), or a salt thereof, or a solvate of the compound or the salt thereof, in the aqueous composition is not particularly limited, and may be determined as appropriate, in consideration of the target disease, or the sex, age, or symptoms of the patient, for example. From the viewpoint of achieving an excellent pharmacological action, however, the content of the compound represented by Formula (1), or a salt thereof, or a solvate of the compound or the salt thereof, is preferably 0.01 to 10 w/v%, more preferably 0.02 to 8 w/v%, and particularly preferably 0.04 to 6 w/v%, calculated as the free form of the compound represented by Formula (1), based on the total volume of the aqueous composition. In particular, when ripasudil is used as the compound represented by Formula (1), from the viewpoint of achieving an excellent pharmacological action, the content of ripasudil, or a salt thereof, or a solvate of ripasudil or the salt thereof, is preferably 0.05 to 5 w/v%, more preferably 0.1 to 3 w/v%, and particularly preferably 0.1 to 2 w/v%, calculated as the free form, based on the total volume of the aqueous composition.

As used herein, the "aqueous composition" means a composition containing at least water, which may be in the form of a liquid (solution or suspension) or a semisolid (ointment), for example. As the water in the composition, purified water, water for injection, or sterile purified water, for example, can be used.

While the content of water in the aqueous composition is not particularly limited, it is preferably 5 mass% or more, more preferably 20 mass% or more, still more preferably 50 mass% or more, even more preferably 90 mass% or more, and particularly preferably 90 to 99.8 mass%.

The aqueous composition can be prepared into various dosage forms in accordance with known methods described in the General Rules for Preparations in the Japanese Pharmacopoeia 16^{th} Edition, for example. While the dosage form is not particularly limited as long as it can be stored in the below-described container, examples of dosage forms include injections, inhalation solutions, eye drops, eye ointments, ear drops, nasal drops, enemas, liquids for external use, sprays, ointments, gels, oral liquids, and syrups. From the viewpoint of advantageously utilizing the pharmacological action of the compound represented by Formula (1), the dosage form is an agent for an eye disease, which specifically is preferably an eye drop or an eye ointment, and is particularly preferably an eye drop.

The aqueous composition may contain, in addition to the components described above, additives used in drugs, quasi drugs, and the like, in accordance with the dosage form. Examples of such additives include inorganic salts, isotonic agents, chelating agents, stabilizers, pH regulators, antiseptics, antioxidants, thickeners, surfactants, solubilizers, suspending agents, cooling agents, dispersants, preservatives, oily bases, emulsion bases, and water-soluble bases.

Specific examples of these additives include ascorbic acid, potassium aspartate, sodium bisulfite, alginic acid, sodium benzoate, benzyl benzoate, epsilon-aminocaproic acid, fennel oil, ethanol, ethylene-vinyl acetate copolymer, sodium edetate, tetrasodium edetate, potassium chloride, calcium chloride hydrate, sodium chloride, magnesium chloride, hydrochloric acid, alkyldiaminoethylglycine hydrochloride solution, carboxyvinyl polymer, dried sodium sulfite, dried sodium carbonate, d-camphor, dl-camphor, xylitol, citric acid hydrate, sodium citrate hydrate, glycerin, gluconic acid, L-glutamic acid, monosodium L-glutamate, creatinine, chlorhexidine gluconate solution, chlorobutanol, sodium dihydrogen phosphate dihydrate, geraniol, sodium chondroitin sulfate, acetic acid, potassium acetate, sodium acetate hydrate, titanium oxide, gellan gum, dibutylhydroxytoluene, potassium bromide, benzododecinium bromide, tartaric acid, sodium hydroxide, polyoxyl 45 stearate, purified lanolin, D-sorbitol, sorbitol solution, sorbic acid, potassium sorbate, taurine, sodium bicarbonate, sodium carbonate hydrate, sodium thiosulfate hydrate, thimerosal, tyloxapol, sodium dehydroacetate, trometamol, concentrated glycerin, mixed tocopherol concentrate, white petrolatum, mentha water, mentha oil, benzalkonium chloride concentrated solution 50, ethyl parahydroxybenzoate, butyl parahydroxybenzoate, propyl parahydroxybenzoate, methyl parahydroxybenzoate, sodium hyaluronate, human serum albumin, hydroxyethyl cellulose, hydroxypropyl cellulose, hypromellose, glacial acetic acid, sodium pyrosulfite, phenylethyl alcohol, glucose, propylene glycol, bergamot oil, benzalkonium chloride, benzalkonium chloride solution, benzyl alcohol, benzethonium chloride, benzethonium chloride solution, borax, boric acid, povidone, polyoxyethylene (200) polyoxypropylene glycol (70), sodium polystyrene sulfonate, polysorbate 80, polyoxyethylene hydrogenated castor oil 60, partially hydrolyzed polyvinyl alcohol, d-borneol, macrogol 4000, macrogol 6000, D-mannitol, anhydrous citric acid, anhydrous sodium monohydrogen phosphate, anhydrous sodium dihydrogen phosphate, methanesulfonic acid, methylcellulose, 1-menthol, monoethanolamine, aluminum monostearate, polyethylene glycol monostearate, eucalyptus oil, potassium iodide, sulfuric acid, oxyquinoline sulfate, liquid paraffin, borneo camphor, phosphoric acid, dibasic sodium phosphate hydrate, potassium dihydrogenphosphate, sodium dihydrogenphosphate, sodium dihydrogenphosphate monohydrate, malic acid, and petrolatum.

Examples of preferred additives include potassium chloride, calcium chloride hydrate, sodium chloride, magnesium chloride, glycerin, acetic acid, potassium acetate, sodium acetate hydrate, tartaric acid, sodium hydroxide, sodium bicarbonate, sodium carbonate hydrate, concentrated glycerin, hydroxyethyl cellulose, hydroxypropyl cellulose, hypromellose, borax, boric acid, povidone, polysorbate 80, polyoxyethylene hydrogenated castor oil, polyethylene glycol monostearate, partially hydrolyzed polyvinyl alcohol, macrogol 4000, macrogol 6000, anhydrous citric acid, anhydrous sodium monohydrogen phosphate, anhydrous sodium dihydrogen phosphate, methylcellulose, monoethanolamine, phosphoric acid, dibasic sodium phosphate hydrate, potassium dihydrogenphosphate, sodium dihydrogenphosphate, sodium dihydrogenphosphate monohydrate, sodium hyaluronate, glucose, and 1-menthol.

The aqueous composition may further contain, in addition to the components described above, other medicinal components in accordance with the target disease and the like. Examples of such medicinal components include α1 receptor blockers including bunazosin, or a salt thereof, or a solvate of bunazosin or the salt thereof, such as bunazosin hydrochloride; α2 receptor agonists including brimonidine, or a salt thereof, or a solvate of brimonidine or the salt thereof, such as brimonidine tartrate, and apraclonidine, or a salt thereof, or a solvate of apraclonidine or the salt thereof; β blockers including carteolol, or a salt thereof, or a solvate of carteolol or the salt thereof, such as carteolol hydrochloride, nipradilol, or a salt thereof, or a solvate or nipradilol or the salt thereof, timolol, or a salt thereof, or a solvate of timolol or the salt thereof, such as timolol maleate, betaxolol, or a salt thereof, or a solvate of betaxolol or the salt thereof, such as betaxolol hydrochloride, levobunolol, or a salt thereof, or a solvate of levobunolol or the salt thereof, such as levobunolol hydrochloride, befunolol, or a salt thereof, or a solvate of befunolol or the salt thereof, and metipranolol, or a salt thereof, or a solvate of metipranolol or the salt thereof; carbonic anhydrase inhibitors including dorzolamide, or a salt thereof, or a solvate of dorzolamide or the salt thereof, such as dorzolamide hydrochloride, brinzolamide, or a salt thereof, or a solvate of brinzolamide or the salt thereof, acetazolamide, or a salt thereof, or a solvate of acetazolamide or the salt thereof, dichlorphenamide, or a salt thereof, or a solvate of dichlorphenamide or the salt thereof, and methazolamide, or a salt thereof, or a solvate of methazolamide or the salt thereof; prostaglandin F2α derivatives including isopropyl unoprostone, or a salt thereof, or a solvate of isopropyl unoprostone or the salt thereof, tafluprost, or a salt thereof, or a solvate of tafluprost or the salt thereof, travoprost, or a salt thereof, or a solvate of travoprost or the salt thereof, bimatoprost, or a salt thereof, or a solvate of bimatoprost or the salt thereof, latanoprost, or a salt thereof, or a solvate of latanoprost or the salt thereof, cloprostenol, or a salt thereof, or a solvate of cloprostenol or the salt thereof, and fluprostenol, or a salt thereof, or a solvate of fluprostenol or the salt thereof; sympathomimetics including dipivefrine, or a salt thereof, or a solvate of dipivefrine or the salt thereof, such as dipivefrine hydrochloride, and epinephrine, or a salt thereof, or a solvate of epinephrine or the salt thereof, such as epinephrine, epinephrine borate, or epinephrine hydrochloride; parasympathomimetics including distigmine bromide, or a salt thereof, or a solvate of distigmine or the salt thereof, pilocarpine, or a salt thereof, or a solvate of pilocarpine or the salt thereof, such as pilocarpine, pilocarpine hydrochloride, or pilocarpine nitrate, and carbachol, or a salt thereof, or a solvate of carbachol or the salt thereof; calcium antagonists including lomerizine, or a salt thereof, or a solvate of lomerizine or the salt thereof, such as lomerizine hydrochloride; and cholinesterase inhibitors including demecarium, or a salt thereof, or a solvate of demecarium or the salt thereof, echothiophate, or a salt thereof or a solvate of echothiophate or the salt thereof, and physostigmine, or a salt thereof, or a solvate of physostigmine or the salt thereof. One or more of these medicinal components can be incorporated.

Preferred as the other medicinal components is one or more selected from the group consisting of latanoprost, nipradilol, dorzolamide, brinzolamide, and timolol, as well as salts thereof.

The pH of the aqueous composition is not particularly limited, but is preferably 4 to 9, more preferably 4.5 to 8, and particularly preferably 5 to 7. The osmotic pressure ratio of the aqueous composition relative to physiological saline is not particularly limited, but is preferably 0.6 to 3, and particularly preferably 0.6 to 2.

As used herein, the "container" means a package for directly storing the aqueous composition. The container is a concept that includes all of the "well-closed container", "hermetic container", and "tight container" defined in the General Notices in the Japanese Pharmacopoeia 16^{th} Edition.

The form of the container is not particularly limited as long as it can house the aqueous composition, and may be selected or set as appropriate, depending on the dosage form, the use of the pharmaceutical preparation, for example. Specific examples of such forms include containers for injections, containers for inhalations, containers for sprays, bottle-shaped containers, tubular containers, containers for eye drops, containers for nasal drops, containers for ear drops, and bag containers.

As used herein, the "container made of polyolefin-based resin" means a container of which at least a portion that contacts the aqueous composition is "made of polyolefin-based resin". Thus, for example, a container in which a polyolefin layer is provided on an inner layer that contacts the aqueous composition, and another resin material or the like is, for example, laminated on an outer side thereof, also corresponds to the "container made of polyolefin-based resin". The polyolefin-based resin used according to the invention is polyethylene or polypropylene. In the case of a copolymer, the mode of polymerization is not particularly limited, and may be random polymerization or block polymerization. Further, the stereoregularity (tacticity) of the polyolefin-based resin is not particularly limited.

Specific examples of such polyolefin-based resins include polyethylene (more specifically, such as low-density polyethylene (including linear low density polyethylene), high-density polyethylene, and medium-density polyethylene), polypropylene, cyclic polyolefins, poly(4-methylpentene), polytetrafluoroethylene, ethylenepropylene copolymer, ethylene-α-olefin copolymer, ethylene-acrylic acid copolymer, ethylene-methacrylic acid copolymer, ethylene-vinylacetate copolymer, and ethylene-ethyl acrylate copolymer. These polyolefin-based resins can be used singly or in combination of two or more. From the viewpoint of suppressing discoloration, the polyolefin-based resin is preferably polyethylene, polypropylene, or a cyclic polyolefin, more preferably polyethylene or polypropylene, and particularly preferably polypropylene.

As used herein, the expression "made of polyolefin-based resin" means that the polyolefin-based resin is included in at least a portion of the material, and "made of polyolefin-based resin" also includes, for example, a mixture of two or more resins (polymer alloy), which are a polyolefin-based resin and other resins.

It is preferred to further mix a substance that prevents transmission of ultraviolet light, such as an ultraviolet absorber or an ultraviolet scattering agent, into the container made of polyolefin-based resin. This improves the stability of the compound represented by Formula (1) with respect to light. Specific examples of such substances will be given below. Examples of ultraviolet scattering agents include titanium oxide and zinc oxide. Examples of ultraviolet absorbers include benzotriazole-based ultraviolet absorbers such as 2-(2H-benzotriazol-2-yl)-p-cresol (for example, Tinuvin P: BASF Corporation), 2-(2H-benzotriazol-2-yl)-4,6-bis(1-methyl-1-phenylethyl)phenol (for example, Tinuvin 234: BASF Corporation), 2-(3,5-di-t-butyl-2-hydroxyphenyl)benzotriazole (for example, Tinuvin 320: BASF Corporation), 2-[5-chloro(2H)-benzotriazol-2-yl]-4-methyl-6-(tert-butyl)phenol (for example, Tinuvin 326: BASF Corporation), 2-(3,5-di-t-butyl-2-hydroxyphenyl)-5-chlorobenzotriazole (for example, Tinuvin 327: BASF Corporation), 2-(2H-benzotriazol-2-yl)-4,6-di-tert-pentylphenol (for example, Tinuvin PA328: BASF Corporation), 2-(2H-benzotriazol-2-yl)-4-(1,1,3,3-tetramethylbutyl)phenol (for example, Tinuvin 329: BASF Corporation), 2,2'-methylenebis[6-(2H-benzotriazol-2-yl)-4-(1,1,3,3-tetramethylbutyl)phenol (for example, Tinuvin 360: BASF Corporation), a reaction product of methyl 3-(3-(2H-benzotriazol-2-yl)-5-tert-butyl-4-hydroxyphenyl)propionate and polyethylene glycol 300 (for example, Tinuvin 213: BASF Corporation), 2-(2H-benzotriazol-2-yl)-6-dodecyl-4-methylphenol (for example, Tinuvin 571: BASF Corporation), 2-(2'-hydroxy-3', 5'-di-t-amylphenyl)benzotriazole, 2-[2'-hydroxy-3'-(3",4",5",6"-tetrahydrophthalimidomethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbers such as 2,2-bis{[2-cyano-3,3-diphenylacryloyloxy]methyl}propane-1,3-diyl=bis(2-cyano-3,3-diphenylacrylate) (for example, Uvinul 3030 FF: BASF Corporation), ethyl 2-cyano-3,3-diphenylacrylate (for example, Uvinul 3035: BASF Corporation), and 2-ethylhexyl 2-cyano-3,3-diphenylacrylate (for example, Uvinul 3039: BASF Corporation); triazine-based ultraviolet absorbers such as 2-(4,6-diphenyl-1,3,5-triazin-2-yl)-5-[(hexyl)oxy]-phenol (for example, Tinuvin 1577 ED: BASF Corporation); benzophenone-based ultraviolet absorbers such as octabenzone (for example, Chimassorb 81: BASF Corporation), 2,2'-dihydroxy-4,4'-dimethoxybenzophenone (for example, Uvinul 3049: BASF Corporation), 2,2'-4,4'-tetrahydrobenzophenone (for example, Uvinul 3050: BASF Corporation), oxybenzone, hydroxymethoxybenzophenonesulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamate-based ultraviolet absorbers such as methyl diisopropylcinnamate, cinoxate, glyceryl mono-2-ethylhexanoate di-p-methoxycinnamate, isopropyl p-methoxycinnamate-diisopropyl cinnamate ester mixture, 2-ethylhexyl p-methoxycinnamate, and benzyl cinnamate; benzoate-based ultraviolet absorbers such as p-aminobenzoic acid, ethyl p-aminobenzoate, glyceryl p-aminobenzoate, amyl p-dimethylaminobenzoate, 2-ethylhexyl p-dimethylaminobenzoate, and ethyl 4-[N,N-di(2-hydroxypropyl)amino]benzoate; salicylate-based ultraviolet absorbers such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris [4-(2-ethylhexyloxycarbonyl)anilino] 1,3,5-triazine; p-hydroxyanisole; 4-tert-butyl-4'-methoxydibenzoylmethane; phenylbenzimidazole sulfonate; and hexyl 2-(4-diethylamino-2-hydroxybenzoyl)benzoate.

Note that when the substance that prevents transmission of ultraviolet light is mixed into the container, the proportion of the substance to be incorporated varies depending on the type or the like of the substance, but it may be 0.001 to 50 mass%, for example, preferably 0.002 to 25 mass%, and particularly preferably about 0.01 to 10 mass%, in the container.

The inside of the container is preferably visible (observable) by the naked eye. When the inside is visible, the following advantages are provided. For example, the presence or absence of any foreign matter can be inspected in the manufacturing steps of the pharmaceutical preparation, and the residual amount of the contents (aqueous composition) can be examined by a user of the pharmaceutical preparation. Visibility may be provided herein through at least a portion of surfaces of the container (for example, even if the side surface of a container for eye drops cannot be seen through due to the presence of a shrinkable film or the like, the container can be determined to be visible if the bottom surface is visible.). If the inside of the container is visible through a portion of surfaces of the container, the aqueous composition inside the container can be thereby examined.

The means for storing the aqueous composition into the container is not particularly limited, and the package may be filled with the aqueous composition using a conventional method, in accordance with the form of the container and the like.

The disease targeted by the pharmaceutical preparation is not particularly limited, and may be selected as appropriate depending on the pharmacological action or the like of the compound represented by Formula (1) .

Specifically, the pharmaceutical preparation can be used, for example, as a prophylactic or therapeutic agent for ocular hypertension or glaucoma, based on the Rho kinase inhibitory action or intraocular pressure-lowering action of the compound represented by Formula (1). More specifically, examples of types of glaucoma include primary open-angle glaucoma, normal-tension glaucoma, hypersecretion glaucoma, acute closed-angle glaucoma, chronic closed-angle glaucoma, plateau iris syndrome, combined mechanism glaucoma, steroid -induced glaucoma, capsular glaucoma, pigmentary glaucoma, amyloid-associated glaucoma, neovascular glaucoma, and malignant glaucoma.

Further, as disclosed in JP-B-5557408, the pharmaceutical preparation can be used as a prophylactic or therapeutic agent for ocular fundus diseases (lesions that mainly develop in the retina and/or choroidea; specifically, for example, hypertensive or arteriosclerotic ocular fundus abnormalities, central retinal artery occlusion, retinal vein occlusion such as central retinal vein occlusion or branch retinal vein occlusion, diabetic retinopathy, diabetic macular edema, diabetic maculopathy, Eales disease, Coats disease and other congenital retinal vascular abnormalities, von Hippel disease, pulseless disease, macular diseases (such as central serous chorioretinopathy, cystoid macular edema, age-related macular degeneration, macular hole, myopic macular degeneration, vitreoretinal interface maculopathy, drug-related maculopathy, or heredomacular degeneration), retinal detachment (rhegmatogenous, tractional, exudative, or the like), retinitis pigmenosa, or retinopathy of prematurity). More preferably, the pharmaceutical preparation can be used as a prophylactic or therapeutic agent for diabetic retinopathy, diabetic macular edema, or age-related macular degeneration.

### [Examples]

The present invention will be described next in more detail with reference to examples; however, the invention is in no way limited to these examples.

In the following test examples, ripasudil monohydrochloride dihydrate can be produced in accordance with the method described in WO2006/057397, for example.

### [Test Example 1] Preservation test No. 1

An aqueous composition of the formulation shown in Table 1 was prepared in accordance with a conventional method, and then placed in a container made of polyethylene (PE), polypropylene (PP), or polyvinyl chloride (PVC) to produce a pharmaceutical preparation.

Each of the resulting pharmaceutical preparations was preserved at 60°C for 3 months, and a color difference (ΔYI) before and after the preservation was measured using a color difference meter (spectrophotometer, CM-700d: Konica Minolta Sensing, Inc.). A ΔYI value of 5 or more was rated as "b", and a ΔYI value of less than 5 was rated as "a".

The results are shown in Table 2.

**[Table 1]**

| Components | Quantity (per 100 mL) |
|---|---|
| Ripasudil Monohydrochloride Dihydrate | 0.4896 g (0.4 g as the free form) |
| Anhydrous Sodium Dihydrogen Phosphate | 0.8 g |
| Sodium Hydroxide | q.s. (pH6.7) |
| Purified Water | Balance |

**[Table 2]**

| Material of Container | ΔYI | Evaluation |
|---|---|---|
| PE | 4.07 | a |
| PP | 4.42 | a |
| PVC | 7.45 | b |

As shown in the results set forth in Table 2, when the aqueous composition containing ripasudil was housed in the container made of polyolefin-based resin, such as polyethylene (PE) or polypropylene (PP), the ΔYI value was reduced even after the aqueous composition was preserved at high temperature for a long period of time; in contrast, the ΔYI value was increased when the aqueous composition was housed in the container made of polyvinyl chloride (PVC).

### [Test Example 2] Preservation test No. 2

An aqueous composition of the formulation shown in Table 3 was prepared in accordance with a conventional method, and then placed in a container made of polyethylene (PE) or polypropylene (PP) to produce a pharmaceutical preparation.

Each of the resulting pharmaceutical preparations was preserved at 60°C for 3 months, and a color difference (ΔYI) before and after the preservation was measured using a color difference meter (spectrophotometer, CM-700d: Konica Minolta Sensing, Inc.). A ΔYI value of 5 or more was rated as "b", and a ΔYI value of less than 5 was rated as "a".

The results are shown in Table 4.

**[Table 3]**

| Components | Quantity (per 100 mL) |
|---|---|
| Ripasudil Monohydrochloride Dihydrate | 0.4896 g(0.4 g as the free form) |
| Boric Acid | 1.2 g |
| Sodium Hydroxide | q.s. (pH6.7) |
| Purified Water | Balance |

**[Table 4]**

| Material of Container | ΔYI | Evaluation |
|---|---|---|
| PE | 4.15 | a |
| PP | 4.64 | a |

As shown in the results set forth in Table 4, even though the formulation of the aqueous composition was changed, when the aqueous composition was stored in the container made of polyolefin-based resin, such as polyethylene (PE) or polypropylene (PP), the ΔYI value was reduced even after preservation at high temperature for a long period of time.

The foregoing results of Test Examples 1 and 2 revealed that when the aqueous composition containing the compound represented by Formula (1) including ripasudil, or a salt thereof, or a solvate of ripasudil or the salt thereof, is stored in a container made of polyolefin-based resin, discoloration is relatively unlikely to occur even after preservation at high temperature for a long period of time, and excellent preservation stability can be achieved.

### [Production Examples 1 to 27]

Pharmaceutical preparations of Production Examples 1 to 27 can be produced by preparing aqueous compositions containing the components in the quantities (amounts (g) per 100 mL of the aqueous composition) shown in Tables 5 to 7 in accordance with a conventional method, and storing them in a container for eye drops made of polyethylene.

**[Table 5]**

| | Productio n Example 1 | Productio n Example 2 | Productio n Example 3 | Productio n Example 4 | Productio n Example 5 | Productio n Example 6 | Productio n Example 7 | Productio n Example 8 | Productio n Example 9 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Sodium Chloride | 0.65 | | | | 0.3 | 0.3 | 0.3 | 0.3 | |
| Glycerin | | 2 | | | 1 | | | 0.5 | 1 |
| Propylene Glycol | | | 2 | | | 1 | | 0.5 | 1 |
| Potassium Chloride | | | | 0.6 | | | 0.3 | | |
| Boric Acid | | | | | | | | | |
| Borax | | | | | | | | | |
| Sodium Dihydrogenphospha te Monohydrate | 0.4 | 0.4 | 0.4 | | | 0.4 | 0.4 | 0.4 | 0.4 |
| Dibasic Sodium Phosphate Hydrate | | | | | | | | q.s. | q.s. |
| Anhydrous Sodium Monohydrogen Phosphate | | | | | | q.s. | q.s. | | |
| Potassium Dihydrogenphospha te | | | | 0.4 | 0.4 | | | | |
| Sodium Hydroxide | q.s. | q.s. | q.s. | q.s. | q.s. | | | | |
| Trometamol | | | | | | | | | |
| Hydrochloric Acid | | | | | | | | | |
| Citric Acid Hydrate | 0.1 | | | | | 0.1 | | | |
| Sodium Acetate Hydrate | | 0.1 | | | | 0.1 | | | |
| Sodium Edetate | | | | 0.1 | | | 0.1 | | |
| Benzalkonium Chloride | 0.001 | 0.005 | | 0.001 | 0.005 | 0.01 | 0.001 | 0.005 | |
| Benzethonium Chloride | | | | | | | | | 0.01 |
| Methyl Parahydroxybenzoa te | | | 0.01 | | | | 0.01 | | |
| Propyl Parahydroxybenzoa te | | | 0.01 | | | | 0.01 | | |
| Chlorobutanol | | | | 0.2 | | | | 0.2 | |
| Polysorbate 80 | 0.3 | | | 0.3 | 0.3 | | | 0.3 | 0.3 |
| Polyoxyethylene Castor Oil 60 | | 0.3 | | 0.3 | | 0.3 | | 0.3 | -0.3 |
| Polyethylene Glycol Monostearate | | | 1.5 | 1.5 | | | 1.5 | | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 7 | 7 | 8 |

**[Table 6]**

| | Productio n Example 10 | Productio n Example 11 | Productio n Example 12 | Productio n Example 13 | Productio n Example 14 | Productio n Example 15 | Productio n Example 16 | Productio n Example 17 | Productio n Example 18 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Sodium Chloride | 0.65 | | | | 0.3 | 0.3 | 0.3 | 0.3 | |
| Glycerin | | 2 | | | 1 | | | 0.5 | 1 |
| Propylene Glycol | | | 2 | | | 1 | | 0.5 | 1 |
| Potassium Chloride | | | | 0.6 | | | 0.3 | | |
| Boric Acid | 1.0 | 1.0 | 1.0 | 1.0 | 1.0 | 1.0 | 1.0 | 1.0 | 1.0 |
| Borax | | | | | q.s. | q.s. | q.s. | q.s. | q.s. |
| Sodium Dihydrogenphospha te Monohydrate | | | | | | | | | |
| Dibasic Sodium Phosphate Hydrate | | | | | | | | | |
| Anhydrous Sodium Monohydrogen Phosphate | | | | | | | | | |
| Potassium Dihydrogen phospha te | | | | | | | | | |
| Sodium Hydroxide | q.s. | q.s. | q.s. | q.s. | | | | | |
| Trometamol | | | | | | | | | |
| Hydrochloric Acid | | | | | | | | | |
| Citric Acid Hydrate | 0.1 | | | | | 0.1 | | | |
| Sodium Acetate Hydrate | | 0.1 | | | | 0.1 | | | |
| Sodium Edetate | | | | 0.1 | | | 0.1 | | |
| Benzalkonium Chloride | 0.001 | 0.005 | | 0.001 | 0.005 | 0.01 | 0.001 | 0.005 | |
| Benzethonium Chloride | | | | | | | | | 0.01 |
| Methyl Parahydroxybenzoa te | | | 0.01 | | | | 0.01 | | |
| Propyl Parahydroxybenzoa te | | | 0.01 | | | | 0.01 | | |
| Chlorobutanol | | | | 0.2 | | | | 0.2 | |
| Polysorbate 80 | 0.3 | | | 0.3 | 0.3 | | | 0.3 | 0.3 |
| Polyoxyethylene Castor Oil 60 | | 0.3 | | 0.3 | | 0.3 | | 0.3 | 0.3 |
| Polyethylene Glycol Monostearate | | | 1.5 | 1.5 | | | 1.5 | | 1.5 |

| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
|---|---|---|---|---|---|---|---|---|---|
| pH | 5 | 5 | 6 | 6 | 6.5 | 6.5 | 7 | 7 | 8 |

**[Table 7]**

| | Productio n Example 19 | Productio n Example 20 | Productio n Example 21 | Productio n Example 22 | Productio n Example 23 | Productio n Example 24 | Productio n Example 25 | Productio n Example 26 | Productio n Example 27 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Sodium Chloride | 0.65 | | | | 0.3 | 0.3 | 0.3 | 0.3 | |
| Glycerin | | 2 | | | 1 | | | 0.5 | 1 |
| Propylene Glycol | | | 2 | | | 1 | | 0.5 | 1 |
| Potassium Chloride | | | | 0.6 | | | 0.3 | | |
| Boric Acid | | | | | | | | | |
| Borax | | | | | | | | | |
| Sodium Dihydrogenphospha te Monohydrate | | | | | | | | | |
| Dibasic Sodium Phosphate Hydrate | | | | | | | | | |
| Anhydrous Sodium Monohydrogen Phosphate | | | | | | | | | |
| Potassium Dihydrogenphospha te | | | | | | | | | |
| Sodium Hydroxide | | | | | | | | | |
| Trometamol | 1.5 | 1.5 | 1.5 | 1.5 | 1.5 | 1.5 | 1.5 | 1.5 | 1.5 |
| Hydrochloric Acid | q.s. | q.s. | q.s. | q.s. | q.s. | q.s. | q.s. | q.s. | q.s. |
| Citric Acid Hydrate | 0.1 | | | | | 0.1 | | | |
| Sodium Acetate Hydrate | | 0.1 | | | | 0.1 | | | |
| Sodium Edetate | | | | 0.1 | | | 0.1 | | |
| Benzalkonium Chloride | 0.001 | 0.005 | | 0.001 | 0.005 | 0.01 | 0.001 | 0.005 | |
| Benzethonium Chloride | | | | | | | | | 0.01 |
| Methyl Parahydroxybenzoa te | | | 0.01 | | | | 0.01 | | |
| Propyl Parahydroxybenzoa te | | | 0.01 | | | | 0.01 | | |
| Chlorobutanol | | | | 0.2 | | | | 0.2 | |
| Polysorbate 80 | 0.3 | | | 0.3 | 0.3 | | | 0.3 | 0.3 |
| Polyoxyethylene Castor Oil 60 | | 0.3 | | 0.3 | | 0.3 | | 0.3 | 0.3 |
| Polyethylene Glycol Monostearate | | | 1.5 | 1.5 | | | 1.5 | | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 7 | 7 | 8 |

### [Production Examples 28 to 54]

Pharmaceutical preparations of Production Examples 28 to 54 can be produced as in Production Examples 1 to 27, except for using a container for eye drops made of polypropylene instead of polyethylene.

### [Production Examples 55 to 81 are not according to the invention]

Pharmaceutical preparations of Production Examples 55 to 81 can be produced as in Production Examples 1 to 27, except for using a container for eye drops made of a cyclic polyolefin (COP) instead of polyethylene.

### [Production Examples 82 to 162]

Pharmaceutical preparations of Production Examples 82 to 162 can be produced in accordance with a conventional method as in Production Examples 1 to 81, except that instead of ripasudil monohydrochloride dihydrate, an equal amount of 4-bromo-5-[[(2S)-2-methyl-1,4-diazepan-1-yl]sulfonyl]isoquinoline is used.

### [Industrial Applicability]

In accordance with the present invention, pharmaceutical preparations having excellent preservation stability can be provided, which can be advantageously used in the pharmaceutical industry, for example.

## Claims

1. A pharmaceutical preparation obtained by storing an aqueous composition comprising a compound represented by Formula (1): 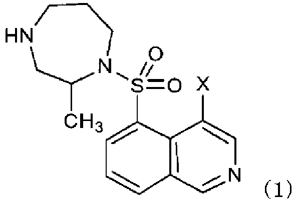 wherein X represents a halogen atom,
or a salt thereof, or a solvate of the compound or the salt thereof, in a container made of polyolefin-based resin, wherein the polyolefin-based resin is polypropylene.

2. The pharmaceutical preparation according to claim 1,
wherein
the compound represented by Formula (1) is ripasudil.

3. Use of a container made of polyolefin-based resin, wherein the polyolefin-based resin is polyethylene or polypropylene, for suppressing discoloration of an aqueous composition comprising a compound represented by Formula (1): 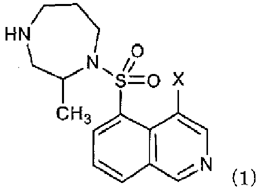 wherein X represents a halogen atom,
or a salt thereof, or a solvate of the compound or the salt thereof.

4. The use according to claim 3, wherein
the compound represented by Formula (1) is ripasudil.

## Patentansprüche

1. Pharmazeutische Zusammensetzung, die durch Aufbewahren einer wässrigen Zusammensetzung, die eine Verbindung der Formel (1) umfasst: 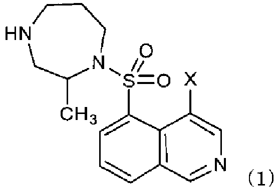 wobei X für ein Halogenatom steht, oder eines Salzes derselben oder eines Solvats der Verbindung oder eines Salz desselben, in einem Behälter aus einem Harz aus Polyolefinbasis erhalten wird, wobei das Harz auf Polyolefinbasis Polypropylen ist.

2. Pharmazeutische Zusammensetzung nach Anspruch 1, wobei die Verbindung der Formel (1) Ripasudil ist.

3. Verwendung eines Behälters aus einem Harz auf Polyolefinbasis, wobei das Harz auf Polyolefinbasis Polyethylen oder Polypropylen ist, um eine Verfärbung einer wässrigen Zusammensetzung, die eine Verbindung der Formel (1) umfasst: 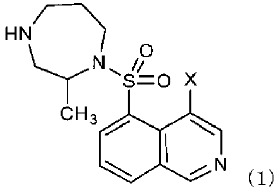 wobei X für ein Halogenatom steht, oder eines Salzes derselben oder eines Solvats der Verbindung oder eines Salzes desselben, zu unterbinden.

4. Verwendung nach Anspruch 3, wobei die Verbindung der Formel (1) Ripasudil ist.

## Revendications

1. Préparation pharmaceutique obtenue par stockage d'une composition aqueuse comprenant un composé représenté par la formule (1) : 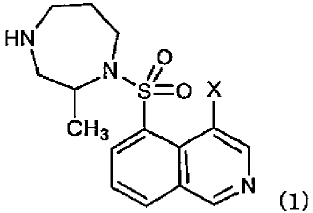 dans laquelle X représente un atome d'halogène,
ou un sel de celui-ci, ou un solvate du composé ou de son sel, dans un récipient fait en une résine à base de polyoléfine, dans laquelle la résine à base de polyoléfine est le polypropylène.

2. Préparation pharmaceutique selon la revendication 1, dans laquelle le composé représenté par la formule (1) est le ripasudil.

3. Utilisation d'un récipient fait en une résine à base de polyoléfine, dans laquelle la résine à base de polyoléfine est le polyéthylène ou le polypropylène, pour réprimer la décoloration d'une composition aqueuse comprenant un composé représenté par la formule (1) : 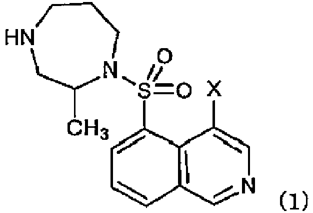 dans laquelle X représente un atome d'halogène,
ou un sel de celui-ci, ou un solvate du composé ou de son sel.

4. Utilisation selon la revendication 3, dans laquelle le composé représenté par la formule (1) est le ripasudil.
